Europäisches Patentamt

European Patent Office

Office européen des brevets

(19)

(11) Veröffentlichungsnummer: **0 069 825**

A2

(12) # EUROPÄISCHE PATENTANMELDUNG

(21) Anmeldenummer: 82102441.1

(22) Anmeldetag: 24.03.82

(51) Int. Cl.³: **A 61 B 6/04**

(30) Priorität: 04.04.81 DE 3113685

(43) Veröffentlichungstag der Anmeldung:
19.01.83 Patentblatt 83/3

(84) Benannte Vertragsstaaten:
AT BE CH FR GB IT LI NL SE

(71) Anmelder: Hildebrandt, Hans-Dietrich, Dr. med.
Hohlesteinweg 16
D-3501 Ahnatal(DE)

(72) Erfinder: Hildebrandt, Hans-Dietrich, Dr. med.
Hohlesteinweg 16
D-3501 Ahnatal(DE)

(74) Vertreter: Freiherr von Schorlemer, Reinfried et al,
Brüder-Grimm-Platz 4
D-3500 Kassel(DE)

(54) Vorrichtung zum Fixieren der Finger bei gehaltenen Röntgenaufnahmen.

(57) Halteapparat zur Herstellung gehaltener Röntgenaufnahmen von Fingern, wobei er ein Traggestell mit einer Führung zum Einschub einer Röntgenkassette aufweist und wobei dicht oberhalb der Führung ein Fixierungsklotz und ein zum Verschwenken des zu untersuchenden Fingers um den Fixierungsklotz bestimmter Schieber angeordnet sind.

Fig. 1.

EP 0 069 825 A2

Halteapparat zur Herstellung gehaltener Röntgenaufnahmen von Fingern

Die Erfindung betrifft einen Halteapparat zur Herstellung gehaltener Röntgenaufnahmen von Fingern, insbesondere Daumen.

Bandrupturen, die sich häufig als Folge von Sportunfällen, aber auch aufgrund anderer Ursachen im Bereich der Daumen oder der Langfinger ergeben, sind nur durch sofortige Operation heilbar. Selbst bei geringstem Verdacht auf das Vorliegen einer Bandruptur muß sich daher der Arzt durch Anfertigen einer Röntgenaufnahme vergewissern, ob eine Bandruptur vorliegt oder nicht.

Die Verifizierung einer Bandruptur an den Fingern erfordert bisher das Auflegen der Hand auf eine Röntgenkassette, das Aufklappen des zu untersuchenden Gelenks und das Fixieren der Hand in dieser Lage durch den behandelnden Arzt oder eine Hilfsperson während der Röntgenaufnahme. Eine Folge davon ist, daß der Arzt bzw. die Hilfsperson trotz der geringen Streustrahlung moderner Röntgenapparate bei jeder Aufnahme eine unkontrollierbare Dosis an Röntgenstrahlung aufnimmt. Bei häufiger Anfertigung derartiger Aufnahmen kann daher die höchstzulässige Strahlendosis überschritten werden.

Der Erfindung liegt daher die Aufgabe zugrunde, einen Halteapparat der eingangs bezeichneten Art zu schaffen, mittels dessen die Finger, insbesondere Daumen, derart gespreizt und gehalten werden können, daß die Anwesenheit eines Arztes in unmittelbarer Nähe des Patienten während der Röntgenaufnahme nicht mehr erforderlich ist.

Zur Lösung dieser Aufgabe sind die kennzeichnenden Merkmale des Anspruchs 1 vorgesehen.

Der erfindungsgemäße Halteapparat ermöglicht das Anlegen
des Daumenballens oder anderer zu fixierender Finger- oder
Daumenteile an den Fixierungsklotz und das gleichzeitige
Aufklappen des zu untersuchenden Gelenks durch Verschwenkung der Daumen- oder Fingerspitze um den Fixierungsklotz.
Da hierbei der zu untersuchende Finger auf der Röntgenkassette zu liegen kommt, während gleichzeitig die übrigen
Finger unter Umgreifen der Kassette unterhalb derselben
angeordnet werden können, kann die vom Arzt mittels des
Fixierungsklotzes und des Schiebers eingestellte Position
vom Patienten selbst aufrechterhalten werden, so daß während der nachfolgenden Röntgenaufnahme kein unterstützendes Halten durch einen Dritten erforderlich ist.

Weitere vorteilhafte Merkmale der Erfindung sind in den
Unteransprüchen gekennzeichnet,

Die Erfindung wird nachfolgend in Verbindung mit der beiliegenden Zeichnung an einem Ausführungsbeispiel näher
erläutert. Es zeigen:

Fig. 1 die perspektivische Ansicht eines erfindungsgemäßen
       Halteapparates;

Fig. 2 eine Draufsicht auf den Halteapparat nach Fig. 1;

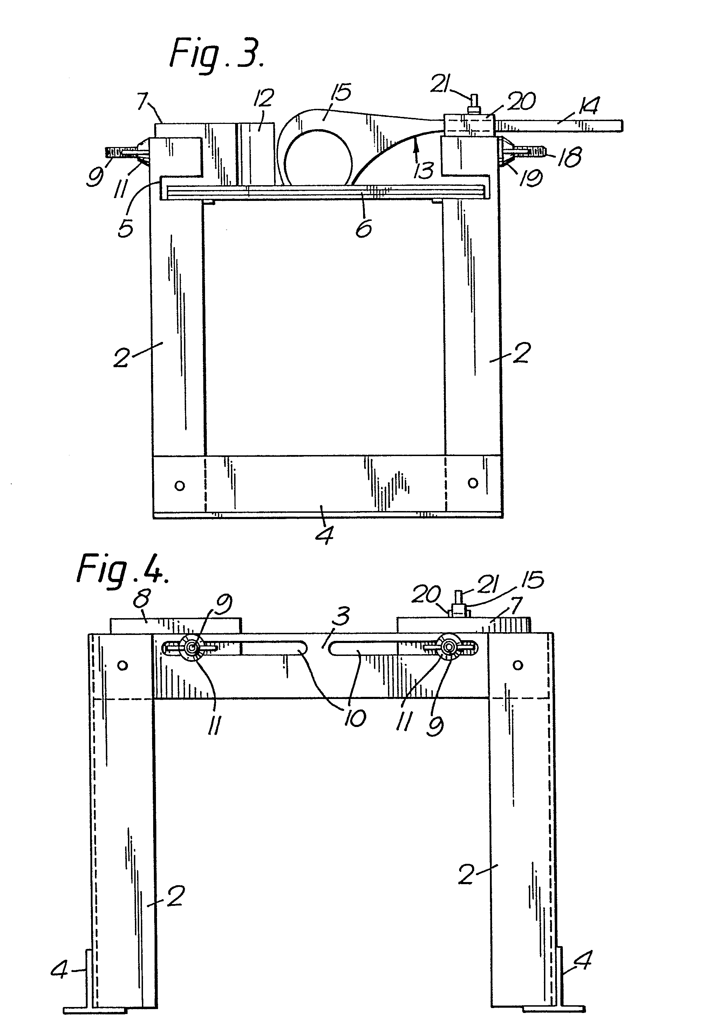

Fig. 3 die Vorderansicht des Halteapparats nach Fig. 1;
       und

Fig. 4 eine Seitenansicht des Halteapparats nach Fig. 1
       mit Blick auf die Fixierungsklötze.

Der erfindungsgemäße Halteapparat zur Herstellung gehaltener Aufnahmen enthält ein Traggestell 1 mit vier Stützen
2, die durch Längsstreben 3 und Querstreben 4 zu einer Baueinheit miteinander verbunden sind. In einem oberen Teil
weist das Traggestell je eine parallel zu einer Längsstrebe 3 angeordnete Führung 5 in Form einer Nut auf, in die eine Röntgenkassette 6 einschiebbar ist, wobei die hinteren
Stützen 2 als Anschlag dienen können. An der vorderen und
hinteren Stirnseite ist das Traggestell 1 zweckmäßig derart
offen, daß die Röntgenkassette 6 von oben und unten erfaßt
bzw. mit einer Hand umgriffen werden kann.

An einer Längsseite des Traggestells 1 sind zwei Fixierungsklötze 7 und 8 für die rechte bzw. linke Hand vorgesehen,
die in nicht näher bezeichneten Führungen geführt sind, parallel zur Längsseite hin- und hergeschoben werden können
und zweckmäßig so dicht oberhalb der Röntgenkassette 6 angeordnet sind, daß diese gerade noch das zum Einschieben
bzw. Herausziehen erforderliche Spiel hat. Zum Anordnen
der Fixierungsklötze 7 und 8 in einer erwünschten Position
ist an diesen beispielsweise je ein Gewindezapfen 9 angebracht, der durch einen parallel zur Längsseite verlaufenden Schlitz 10 in der einen Längsstrebe 3 ragt und auf den
von außen her eine Flügelmutter 11 aufgeschraubt ist. Dadurch ist einerseits der Verschiebeweg der Fixierungsklötze auf die Länge der Schlitze 10 begrenzt, während andererseits jeder Fixierungsklotz 7,8 in jeder möglichen Position mittels der Flügelmutter 11 festgelegt werden kann.
Außerdem ist die Anordnung zweckmäßig so getroffen, daß
sich beide Fixierungsklötze 7 und 8 jeweils bis zur zugehörigen Stirnseite des Traggestells 1 verschieben und dort
gegen die als Anschläge wirksamen oberen Enden der Stützen 2
legen lassen.

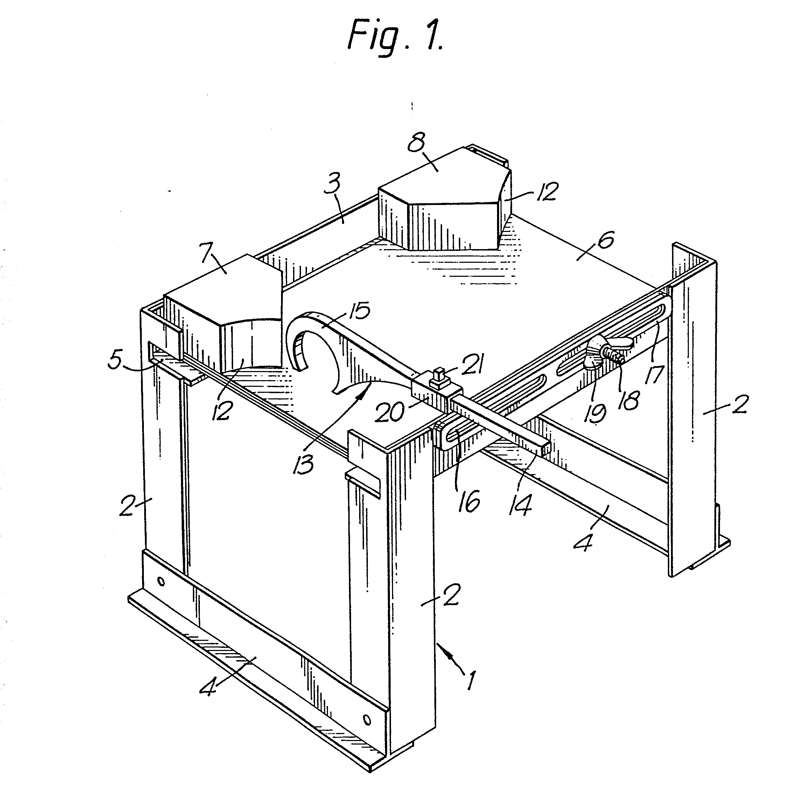

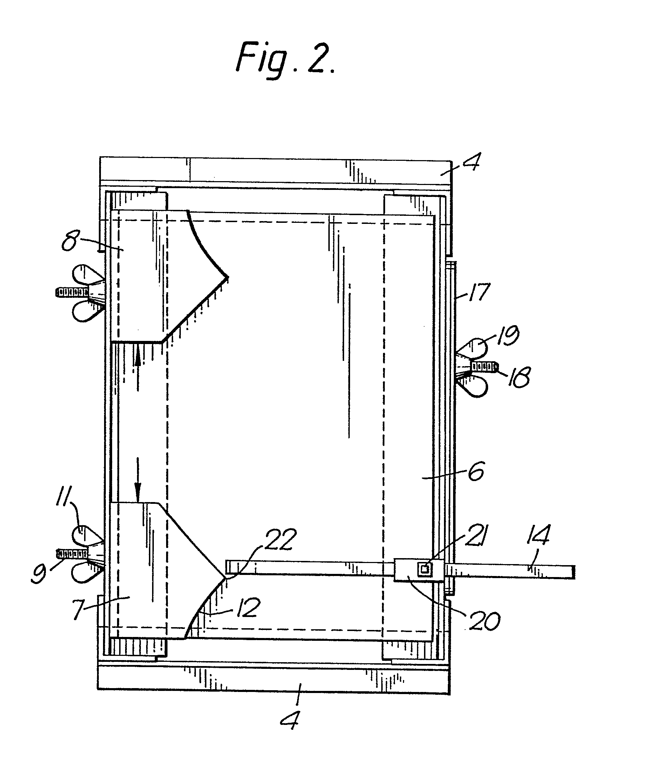

Gemäß Fig. 1 und 2 weist jeder Fixierungsklotz 7 bzw. 8
auf seiner Innenseite eine bogenförmige Anlagefläche 12
auf, an welche der zu fixierende Fingeranteil angelegt
wird und die jeweils der vorderen bzw. hinteren Stirnseite des Traggestells zugewandt ist, jedoch einen Winkel
mit dieser bildet, wie insbesondere aus Fig. 2 ersichtlich
ist. Im übrigen können die Fixierungsklötze 7 und 8 symmetrisch zur quer verlaufenden Mittelebene des Traggestells
ausgebildet sein.

In jeder Anlagefläche 12 kann, was nicht dargestellt ist, außerdem je eine längliche Aushöhlung zur teilweisen Aufnahme des Daumenballens ausgebildet sein, deren Form an die Anatomie eines Daumenballens durchschnittlicher Größe angepaßt ist und in Längsrichtung der Anlagefläche 12 verläuft, um dem anzulegenden Daumenballen einen zusätzlichen Halt zu geben.

An der anderen Längsseite des Traggestells 1 ist ein Schieber 13 gelagert, der aus einem länglichen Stab 14 besteht, dessen Vorderende ein gabel- bzw. greiferförmig ausgebildetes, zur Kassette 6 hin geöffnetes Element 15 trägt. Zur Lagerung des Schiebers 13 ist eine an der Außenseite der anderen Längsstrebe 3 angeordnete, einen Längsschlitz 16 aufweisende Platte 17 vorgesehen, die in an sich beliebiger Weise längsverschiebbar am Traggestell 1 gelagert ist. Der Längsschlitz 16 ist von einem an der Längsstrebe 3 befestigten Gewindezapfen 18 durchragt, der einerseits den Verschiebeweg der Platte 17 begrenzt und andererseits zur Aufnahme einer Flügelmutter 19 (Fig. 2) dient, mittels derer die Platte 17 in jeder erwünschten Position arretiert werden kann.

Gemäß Fig. 1 ist an der Platte 17 eine hülsenförmige Führung 20 für den Stab 14 des Schiebers 13 befestigt, in welcher der Stab 14 senkrecht zur Längsrichtung des Traggestells 1 hin- und hergeschoben werden kann. Zum Festlegen des Stabs 14 in der erwünschten Position dient eine die Führung 20 durchragende Feststellschraube 21, die beispielsweise ein Betätigungsteil nach Art einer Flügelmutter aufweist.

Die Wirkungsweise des beschriebenen Halteapparats ist wie folgt:Zur Anfertigung einer gehaltenen Aufnahme beispielsweise des Daumens einer rechten Hand wird zunächst eine Röntgenkassette 6 in die Führung 5 eingeschoben und dann die Kassette so mit der Hand umfaßt, daß der zu untersuchende Daumen auf der Kassette 6, die übrige Hand dagegen unter der Kassette angeordnet ist. Anschließend wird der
Fixierungsklotz 7 entsprechend der Größe und Lage des Daumenballens in die erforderliche Position gebracht und dort
mittels der Flügelmutter 10 festgelegt. Danach wird der Daumenballen fest gegen die Anlagefläche 12 gedrückt, das
nach unten weisende, gabelförmige Element 15 des Schiebers
13 um das freie Ende des Daumens gelegt und die Platte 17
mittels der Flügelmutter 19 in der erwünschten Lage fixiert.
Schließlich wird der Schieber 13 in der zu den Querstreben
4 parallelen Richtung vorgeschoben. Hierdurch wird das Daumenende um die aus Fig. 2 ersichtliche Hinterkante 22 des
Fixierungsklotzes 7 verschwenkt und das Daumengelenk in der
für die Röntgenaufnahme erforderlichen Weise freigelegt.
Beim Erreichen der erwünschten Position wird die Befestigungsschraube 21 angezogen, wodurch der Daumen für die
nachfolgende, von oben her erfolgende Röntgenaufnahme
vorbereitet ist. Ein Verrutschen des Daumens oder der
ganzen Hand wird dadurch vermieden, daß der Patient die
Röntgenkassette 6 fest mit der Hand umfaßt. Alternativ
kann ein zweiter, nicht dargestellter Schieber vorgesehen
sein, um den Daumenballen an einer vor der Hinterkante 22
gelegenen Stelle in fester Anlage an der Anlagefläche 12
zu halten.

Auf entsprechende Weise kann eine Aufnahme des Daumens der
linken Hand angefertigt werden, indem die Kassette 6 von
der anderen Stirnseite des Traggestells 1 her umfaßt und
der Daumenballen gegen die Anlagefläche 12 des anderen
Fixierungsklotzes 8 gelegt wird.

Da in beiden Fällen jeweils nur eine Hälfte der Röntgenkassette 6 benötigt wird, empfiehlt es sich, beim Anfertigen einer Aufnahme den jeweils nicht benötigten Teil durch
Auflage einer Bleiplatte abzudecken. Auf diese Weise ist
es möglich, sowohl die kranke Hand als auch zu Kontrollzwecken die gesunde Hand eines Patienten mit einer und
derselben Röntgenkassette 6 aufzunehmen.

0069825

Die Erfindung ist nicht auf das beschriebene Ausführungsbeispiel beschränkt, das viele Abwandlungen gestattet. Dies
gilt vor allem für die verschiedenen Führungen und Verstellorgane der Kassette 6, der Fixierungsklötze 7,8 und der Schieber 13. Anstelle der Flügelmuttern 10 und 19 sowie der Befestigungsschraube 21 können andere Befestigungselemente,
vorzugsweise Schnellverschlußelemente vorgesehen sein. Weiterhin könnten auswechselbare Fixierungsklötze vorgesehen
werden, wenn sich dies für die Fixierung extrem großer oder
kleiner Daumen oder Langfinger als zweckmäßig erweisen sollte.
Auch die Form des Schiebers ist nicht auf die dargestellte
Form beschränkt.

Weiterhin ist es nicht erforderlich, zwei Fixierungsklötze
am Traggestell zu lagern. Vielmehr könnte für Aufnahmen der
rechten bzw. linken Hand je ein gesonderter Halteapparat
vorgesehen sein. Gemäß einer weiteren Ausführungsform können
die Fixierungsklötze und Schieber auswechselbar und je nach
Bedarf auf der einen oder anderen Längsseite eines und desselben Traggestells montiert werden. Hierbei könnte der Fixierungsklotz derart keilförmig ausgebildet sein, daß seine
eine Keilfläche, z.B. die Anlagefläche 12, für Aufnahmen
der einen Hand, eine andere, in Fig. 1 hintere Keilfläche
dagegen nach Drehung um 180° für Aufnahmen der anderen Hand
geeignet ist, während die Keilspitze in beiden Fällen als
Schwenkkante dient, die auch abgerundet sein könnte. Außerdem können die Fixierungsklötze 7 und 8 eine andere als
die dargestellte Form besitzen, sofern nur die Möglichkeit
besteht, den Daumen oder Finger um eine vom Fixierungsklotz
vorgegebene Schwenkkante oder dgl. in der Weise zu verschwenken, wie es für die Röntgenaufnahme erforderlich ist.

Um ein Ausweichen der gesamten Hand nach hinten zu vermeiden
bzw. diese in der erwünschten Aufnahmeposition zu fixieren,
kann in Weiterbildung der Erfindung vorgesehen sein, an den
Rändern der Stirnseiten des Traggestells 1, z.B. im Bereich
der Stützen 2, schwenkbare oder klappbare, vorzugsweise flexible
Bügel oder Gurte anzubringen, die über die Hand gelegt und am
jeweils gegenüberliegenden Rand mittels Schnappverbindungen,
Druckknöpfen oder dgl. befestigt werden können.

Bei einer bevorzugten Ausführungsform der Erfindung ist weiterhin ein Befestigungsorgan für die Kassette 6 vorgesehen, das die Kassette nach ihrem Einschub in die Führung 5 gegen Herausrutschen sichert. Das Befestigungsorgan besteht beispielsweise aus einem an der Einschubseite der Führung 5 angebrachten Riegel. Dadurch wird verhindert, daß die Kassette herausrutscht, wenn sie auf der der Einschubseite gegenüber liegenden Seite von der Hand umfaßt und dabei ein gewisser Druck auf sie ausgeübt wird.

Schließlich ist dem Schieber 13 vorzugsweise eine Skala zugeordnet, die eine exakte Positionierung des Schiebers innerhalb der Hülse 20 ermöglicht. Bei der praktischen Anwendung des Halteapparats wird dann zuerst das gesunde Gelenk der einen Hand "aufgeklappt" und die hierfür erforderliche Stellung des Schiebers 13 an der Skala abgelesen. Anschließend wird das entsprechende verletzte und somit schmerzende Gelenk der anderen Hand untersucht. Der vorher abgelesene Skalenwert gibt dabei einen Anhaltspunkt dafür, welche Stellung des Schiebers überschritten werden muß, um eine eventuell vorliegende Verletzung überhaupt nachweisen zu können. Ohne die Skala könnte das individuelle Schmerzempfinden des Patienten möglicherweise Anlaß dazu geben, das schmerzende Gelenk nicht ausreichend weit aufzuklappen.

Patentanwa**0 069825**
Diplom-Physiker
**Reinfried Frhr. v. Schorlemer**

D-3500 Kassel
Brüder-Grimm-Platz 4
Telefon (0561) 153 35

D 5110

Dr. Hans-Dietrich Hildebrandt, 3501 Ahnatal

Ansprüche

1) Halteapparat zur Herstellung gehaltener Röntgenaufnahmen
von Fingern, dadurch gekennzeichnet, daß er ein Traggestell
(1) mit einer Führung (5) zum Einschub einer Röntgenkassette (6)
aufweist und daß dicht oberhalb der Führung (5) ein Fixierungsklotz (7) und ein zum Verschwenken des zu untersuchenden Fingers um den Fixierungsklotz (7) bestimmter Schieber (13)
angeordnet sind.

2) Halteapparat nach Anspruch 1, dadurch gekennzeichnet,
daß das Traggestell (1) zwei einander gegenüber liegende
Längsseiten aufweist und daß der Fixierungsklotz (7) an
der einen Längsseite, der Schieber (13) dagegen an der
anderen Längsseite befestigt ist.

3) Halteapparat nach Anspruch 1 oder 2, dadurch gekennzeichnet, daß der Fixierungsklotz (7) an seiner Innenseite eine bogenförmige Anlagefläche (12) für den zu fixierenden Fingeranteil einer Hand aufweist.

4) Halteapparat nach Anspruch 3, dadurch gekennzeichnet,
daß ein zweiter, an derselben Längsseite des Traggestells
(1) angeordneter und symmetrisch ausgebildeter Fixierungsklotz (8) mit einer Anlagefläche (12) für den zu fixierenden Fingeranteil der anderen Hand vorgesehen ist.

5) Halteapparat nach einem der Ansprüche 1 bis 4, dadurch gekennzeichnet, daß der Fixierungsklotz (7,8) zwecks Anpassung an verschiedene Handgrößen verschiebbar am Traggestell (1) gelagert ist.

6) Halteapparat nach einem der Ansprüche 2 bis 5, dadurch gekennzeichnet, daß der Fixierungsklotz (7,8) parallel zu den Längsseiten des Traggestells (1) verschiebbar und feststellbar gelagert ist.

7) Halteapparat nach einem der Ansprüche 3 bis 6, dadurch gekennzeichnet, daß die Anlagefläche (12) eine anatomiegerechte Aushöhlung für einen Daumenballen aufweist.

8) Halteapparat nach einem der Ansprüche 1 bis 7, dadurch gekennzeichnet, daß der Schieber (13) senkrecht zu den Längsseiten des Traggestells (1) verschiebbar und feststellbar gelagert ist.

9) Halteapparat nach einem der Ansprüche 2 bis 8, dadurch gekennzeichnet, daß der Schieber (13) zwecks Anpassung an verschiedene Fingergrößen parallel zu den Längsseiten des Traggestells (1) verschiebbar und feststellbar gelagert ist.

10) Halteapparat nach einem der Ansprüche 6 bis 9, dadurch gekennzeichnet, daß zur Feststellung des Fixierungsklotzes (7,8) und des Schiebers (13) Schnellverschlußelemente (11, 19,21) vorgesehen sind.

11) Halteapparat nach einem der Ansprüche 1 bis 10, dadurch gekennzeichnet, daß der Schieber (13) an seinem zum Verschwenken des Fingers bestimmten Ende ein greifer- bzw. gabelförmig ausgebildetes Element (15) aufweist.

12) Halteapparat nach einem der Ansprüche 4 bis 11, dadurch gekennzeichnet, daß der Verschiebeweg des Schiebers (13) parallel zu den Längsseiten des Traggestells (1) dem Abstand der beiden Fixierungsklötze (7,8) entspricht.

# Fig. 1.

# Fig. 2.

Fig. 3.

Fig. 4.